# EUROPÄISCHE PATENTSCHRIFT

(11) **EP 4 162 131 B1**
(45) Veröffentlichungstag und Bekanntmachung des Hinweises auf die Patenterteilung: **02.07.2025**
(21) Anmeldenummer: 21730167.0
(22) Anmeldetag: 28.05.2021
(51) Int. Cl.: E05D 7/10, A61M 1/14, A61M 60/113, A61M 60/279, A61M 60/37, A61M 60/835, A61M 60/90

(54) **MEDIZINISCHES GERÄT ZUR EXTRAKORPORALEN BLUTBEHANDLUNG**
MEDICAL APPLIANCE FOR EXTRACORPOREAL BLOOD TREATMENT
DISPOSITIF MÉDICAL POUR TRAITEMENT EXTRACORPOREL DU SANG

(30) Priorität: 03.06.2020 DE 102020206873
(43) Veröffentlichungstag der Anmeldung: 12.04.2023
(73) Patentinhaber: B. Braun Avitum AG, 34212 Melsungen (DE)
(72) Erfinder: HORSCHKE, Sebastian, 37236 Hessisch Lichtenau (DE); JAKOBI, Marco, 37318 Uder (DE)
(74) Vertreter: Patentanwälte Ruff, Wilhelm, Beier, Dauster & Partner mbB
(86) Internationale Anmeldenummer: PCT/EP2021/064379
(87) Internationale Veröffentlichungsnummer: WO 2021/244973

(56) Entgegenhaltungen:
- EP-A1- 2 679 752
- DE-A1- 2 532 484
- KR-U- 20110 000 695
- US-A1- 2002 029 793
- US-A1- 2013 111 818

## Beschreibung

Die Erfindung betrifft ein medizinisches Gerät zur extrakorporalen Blutbehandlung, aufweisend eine Peristaltikpumpe mit einem Pumpengehäuse, einen Deckel und eine Schwenklageranordnung, mittels derer der Deckel an dem Pumpengehäuse und um eine Schwenkachse relativ zu dem Pumpengehäuse schwenkbeweglich gelagert ist, wobei der Deckel wenigstens zwischen einer Abdeckstellung, in welcher der Deckel das Pumpengehäuse abdeckt, und einer Freigabestellung, in welcher der Deckel das Pumpengehäuse freigibt, schwenkbeweglich ist, und wobei die Schwenklageranordnung wenigstens zwei entlang der Schwenkachse voneinander beabstandet angeordnete Schwenklager aufweist.

Ein derartiges medizinisches Gerät ist in Form eines Dialysegeräts zur extrakorporalen Blutbehandlung aus der EP 2 679 752 A1 bekannt. Das Dialysegerät weist eine Peristaltikpumpe mit einem Pumpengehäuse und einem Pumpendeckel auf. Der Pumpendeckel ist mittels einer Schwenklageranordnung an dem Pumpengehäuse gelagert um eine Schwenkachse relativ zu dem Pumpengehäuse zwischen einer Abdeckstellung und einer Freigabestellung schwenkbeweglich. In der Abdeckstellung ist das Pumpengehäuse mittels des Pumpendeckels abgedeckt. In der Freigabestellung gibt der Pumpendeckel das Pumpengehäuse frei. Die Schwenklageranordnung des bekannten Dialysegeräts weist zwei entlang der Schwenkachse voneinander beabstandet angeordnete Schwenklager auf, die als Schwenkgelenke bezeichnet werden und jeweils einen ersten und einen zweiten Gelenkabschnitt aufweisen. Die zweiten Gelenkabschnitte sind jeweils an dem Pumpengehäuse befestigt und hierzu in senkrecht zur Schwenkachse erstreckte Sackbohrungen eingebracht. Zur Befestigung des Pumpendeckels an den Schwenkgelenken ist ein Scharnierstift vorgesehen, der durch eine parallel zur Schwenkachse durch den Pumpendeckel erstreckte Scharnierbohrung gesteckt und einends an einem hierfür vorgesehenen Abschnitt des Pumpendeckels formschlüssig befestigt ist.

Aufgabe der Erfindung ist es, ein medizinisches Gerät der eingangs genannten Art bereitzustellen, das gegenüber dem Stand der Technik einen vereinfachten Aufbau aufweist und insbesondere eine vereinfachte Demontage und Montage des Deckels an dem Gehäusebauteil ermöglicht.

Diese Aufgabe wird dadurch gelöst, dass die Schwenklager jeweils ein Lagerelement und eine Lagerelementaufnahme aufweisen, wobei die Lagerelemente in die jeweilige Lagerelementaufnahme eingreifen und relativ zu derselben um die Schwenkachse schwenkbeweglich und entlang der Schwenkachse begrenzt linearbeweglich sind, wodurch der Deckel entlang der Schwenkachse relativ zu dem Gehäusebauteil linearbeweglich verlagerbar ist zwischen einer Demontagestellung, in welcher wenigstens eines der Lagerelemente außer Eingriff mit der jeweiligen Lagerelementaufnahme ist, und einer Lagerstellung, in welcher beide Lagerelemente in Eingriff mit der jeweiligen Lagerelementaufnahme sind, und wobei eine Federanordnung vorgesehen ist, mittels derer der Deckel entlang der Schwenkachse federnd gelagert und in Richtung der Lagerstellung relativ zu dem Gehäusebauteil elastisch vorgespannt ist. Durch die erfindungsgemäße Lösung wird ein vereinfachter Aufbau des medizinischen Geräts erreicht. Insbesondere wird eine gegenüber dem Stand der Technik deutlich vereinfachte Demontage und Montage des Deckels ermöglicht. Denn durch die begrenzte Linearbeweglichkeit der Lagerelemente in den Lagerelementaufnahmen ist der Deckel nicht lediglich um die Schwenkachse, sondern auch begrenzt axial entlang derselben beweglich. Hierdurch können die Lagerelemente zur Demontage des Deckels außer Eingriff mit der jeweiligen Lagerelementaufnahme gebracht werden. Dies geschieht vereinfacht ausgedrückt durch eine einfache seitliche Linearbewegung des Deckels relativ zu dem Pumpengehäuse. Um zu verhindern, dass die Lagerelemente ungewollt außer Eingriff mit der jeweiligen Lagerelementaufnahme gelangen, beispielsweise bei einer Schwenkbewegung des Deckels, ist die Federanordnung vorgesehen. Diese spannt den Deckel relativ zu dem Pumpengehäuse elastisch federnd in Richtung der Lagerstellung. Hierdurch sind die Lagerelemente sicher in Eingriff mit der jeweiligen Lagerelementaufnahme gehalten. Der Deckel ist mittels der Federanordnung entlang der Schwenkachse, d.h. axial, federnd an dem Gehäusebauteil gelagert. Die Schwenklageranordnung ist mit dem Deckel und mit dem Gehäusebauteil wirkverbunden. In einer Ausgestaltung sind die Lagerelemente jeweils an dem Deckel angeordnet und gemeinsam mit diesem relativ zu dem Gehäusebauteil beweglich. In diesem Fall sind die Lagerelementaufnahmen an dem Gehäusebauteil angeordnet. Alternativ können die Lagerelemente an dem Gehäusebauteil angeordnet sein. In diesem Fall sind die Lagerelementaufnahmen an dem Deckel angeordnet und gemeinsam mit diesem beweglich. Weiter alternativ können eines der Lagerelemente am Deckel und ein weiteres der Lagerelemente am Gehäusebauteil angeordnet sein. In diesem Fall ist dementsprechend eine der Lagerelementaufnahmen am Gehäusebauteil und eine weitere der Lagerelementaufnahmen am Deckel angeordnet. In einer Ausgestaltung sind die Lagerelemente einstückig an dem Deckel und/oder an dem Gehäusebauteil ausgebildet und bilden somit einen einstückigen Funktionsabschnitt des entsprechenden Bauteils. Alternativ sind die Lagerelemente als separate Bauteile gefertigt und mit dem Deckel und/oder dem Gehäusebauteil zusammengefügt. Weiter alternativ ist eines der Lagerelemente einstückig an dem Deckel und/oder an dem Gehäusebauteil ausgebildet und ein weiteres der Lagerelemente ist als separates Bauteil gefertigt. Entsprechendes gilt sinngemäß hinsichtlich einer einstückigen und/oder separaten Ausbildung der Lagerelementaufnahmen. Die Lagerelemente sind vorzugsweise jeweils als Lagerzapfen, -bolzen und/oder -pin gestaltet. Die Lagerelementaufnahmen sind vorzugsweise hierzu komplementär jeweils als Bohrung in Form einer Zapfen-, Bolzen- und/oder Pinaufnahme gestaltet. Die Lagerelemente und die Lagerelementaufnahmen wirken vorzugsweise gleitbeweglich zusammen. Zur besagten axial federnden Lagerung und Vorspannung des Deckels mittels der Federanordnung weist diese in einer Ausgestaltung ein zwischen dem Deckel und dem Gehäusebauteil wirksames Federelement auf. Alternativ kann die axial federnde Lagerung und Vorspannung durch eine wenigstens abschnittsweise federelastische Gestaltung des Deckels, des Gehäusebauteils, wenigstens eines der Lagerelemente und/oder wenigstens einer der Lagerelementaufnahmen bewirkt sein. Die federelastische Gestaltung kann hierbei insbesondere durch weichelastische Werkstoffeigenschaften und/oder durch eine elastisch nachgiebige Formgebung des entsprechenden Bauteils erreicht werden. Erfindungsgemäß ist das Gehäusebauteil ein Pumpengehäuse einer Peristaltikpumpe des medizinischen Geräts. Der Deckel kann in diesem Fall auch als Pumpendeckel bezeichnet werden.

In Ausgestaltung der Erfindung weist die Federanordnung wenigstens ein Federelement auf, das einends an einem der Lagerelemente angreift und andernends an dem Deckel oder an dem Gehäusebauteil wenigstens mittelbar abgestützt ist, oder die Federanordnung ist durch einen elastisch formnachgiebigen Wandabschnitt des Deckels gebildet, an welchem Wandabschnitt eines der Lagerelemente oder eine der Lagerelementaufnahmen ausgebildet ist. Das Federelement wirkt einer Linearbewegung des Deckels entlang der Schwenkachse in Richtung der Demontagestellung federelastisch entgegen. Mit anderen Worten ausgedrückt, spannt bzw. drückt das Federelement den Deckel relativ zu dem Gehäusebauteil in Richtung der Lagerstellung. Hierzu greift das Federelement einends an dem Lagerelement und andernends an dem Deckel oder dem Gehäusebauteil an. Sofern das Lagerelement am Deckel angeordnet ist, greift das Federelement andernends am Deckel an. Sofern das Lagerelement am Gehäusebauteil angeordnet ist, greift das Federelement andernends ebenfalls am Gehäusebauteil an. Alternativ ist die Federanordnung durch den besagten formnachgiebigen Wandabschnitt des Deckels gebildet. Bei einer Verlagerung des Deckels ausgehend von der Lagerstellung in die Demontagestellung wird der Wandabschnitt elastisch deformiert. Die aus der elastischen Deformation resultierende Federkraft wirkt der Verlagerung des Deckels in die Demontagestellung entgegen. Die elastische Formnachgiebigkeit des Wandabschnitts kann durch eine entsprechende Werkstoffwahl und/oder Gestaltung des Wandabschnitts erreicht sein. Beispielsweise kann der Wandabschnitt vergleichsweise dünnwandig sein. Alternativ oder zusätzlich kann der Wandabschnitt aus einem gegenüber übrigen Bauteilen und/oder Abschnitten des medizinischen Geräts vergleichsweise nachgiebigen Werkstoff gefertigt sein.

Das entsprechende Lagerelement oder die entsprechende Lagerelementaufnahme ist vorzugsweise einstückig an dem formnachgiebigen Wandabschnitt ausgebildet.

In weiterer Ausgestaltung der Erfindung ist eine mit dem Deckel und dem Gehäusebauteil wirkverbundene Stützanordnung vorgesehen, mittels derer der Deckel entgegen einer in die Demontagestellung gerichteten Linearbewegung an dem Gehäusebauteil formschlüssig abstützbar ist, wobei die Abstützung wenigstens in der Abdeckstellung bewirkt ist, und wobei die Abstützung in einer definierten Schwenkstellung des Deckels für dessen Demontage aufgehoben ist. Die Stützanordnung wirkt einer ungewollten Demontage des Deckels in der Abdeckstellung und/oder beim Verschwenken um die Schwenkachse entgegen. Hierzu bewirkt die Stützanordnung wenigstens in der Abdeckstellung einen entlang der Schwenkachse wirksamen und somit axialen Formschluss zwischen dem Deckel und dem Gehäusebauteil. Dieser axiale Formschluss wirkt in der Abdeckstellung und blockiert eine axiale Linearbewegung des Deckels in die Demontagestellung. Demgegenüber ist der axiale Formschluss in der definierten Schwenkstellung aufgehoben. Hierdurch kann der Deckel axial entlang der Schwenkachse - und entgegen der Federkraft der Federanordnung - in die Demontagestellung verlagert werden. Die Stützanordnung ist vorzugsweise durch einstückig an dem Deckel und/oder dem Gehäusebauteil ausgebildete Funktionsabschnitte gebildet. Alternativ oder zusätzlich kann die Stützanordnung separate Bauteile aufweisen, die mit dem Deckel und/oder dem Gehäusebauteil zusammengefügt sein können. Die Abstützung ist zwischen axial gegenüberliegenden Bauteilen und/oder Abschnitten des Deckels und des Gehäusebauteils bewirkt.

In weiterer Ausgestaltung der Erfindung weist die Stützanordnung wenigstens ein an dem Deckel angeordnetes Stützelement auf, welches wenigstens in der Abdeckstellung entlang der Schwenkachse an einem Stützabschnitt des Gehäusebauteils formschlüssig abgestützt ist, und welches in der definierten Schwenkstellung des Deckels um die Schwenkachse von dem Stützabschnitt weggeschwenkt und somit nicht an demselben abstützbar ist. Dies ermöglicht einen konstruktiv besonders einfachen Aufbau der Stützanordnung. Das Stützelement ist vorzugsweise einstückig an dem Deckel ausgebildet. Die Abstützung ist vorzugsweise ausschließlich in der definierten Schwenkstellung aufgehoben.

In weiterer Ausgestaltung der Erfindung ist die definierte Schwenkstellung die Freigabestellung oder eine ausgehend von der Freigabestellung weitergehend um die Schwenkachse verschwenkte Schwenkstellung des Deckels. Die erste Variante gestattet die Demontage des Deckels in der Freigabestellung. Demgegenüber ist bei der zweiten Varianten die Demontage des Deckels nicht etwa in der Freigabestellung, sondern in der weitergehend verschwenkten Schwenkstellung möglich. Dies wirkt einem unbeabsichtigten Demontieren des Deckels in der Freigabestellung entgegen. Hierdurch kann eine verbesserte Anwendungssicherheit erreicht werden.

In weiterer Ausgestaltung der Erfindung weist die Stützanordnung wenigstens ein an dem Deckel angeordnetes Gegenstützelement auf, das wenigstens in der Abdeckstellung entlang der Schwenkachse und entgegengesetzt zu dem Stützelement an einem Gegenstützabschnitt des Gehäusebauteils formschlüssig abgestützt ist. Hierdurch kann insbesondere eine verbesserte, nämlich beidseitige, Abstützung des Deckels in der Abdeckstellung erreicht werden. In einer Ausgestaltung der Erfindung ist das Gegenstützelement auch in der Freigabestellung und/oder in der weitergehend verschwenkten Schwenkstellung des Deckels an dem Gegenstützabschnitt abgestützt. Dies bietet insbesondere eine verbesserte seitliche Führung des Deckels beim Verschwenken um die Schwenkachse. Das Gegenstützelement ist vorzugsweise einstückig an dem Deckel ausgebildet.

In weiterer Ausgestaltung der Erfindung ist eine mit dem Deckel und dem Gehäusebauteil wirkverbundene Halteanordnung vorgesehen, mittels derer der Deckel entgegen einer in die Abdeckstellung gerichteten Schwenkbewegung in der Freigabestellung gehalten ist. Somit verhindert die Halteanordnung, dass der Deckel ungewollt von der Freigabe- in die Abdeckstellung zurückschwenkt, beispielsweise unter Einwirkung der Schwerkraft. Die Halteanordnung ist vorzugsweise durch einstückig an dem Deckel und/oder dem Gehäusebauteil ausgebildete Funktionsabschnitte gebildet. Alternativ oder zusätzlich kann die Halteanordnung separat gefertigte Bauteile aufweisen, die mit dem Deckel oder dem Gehäusebauteil zusammengefügt sind. Die Halteanordnung wirkt in der Freigabestellung vorzugsweise formschlüssig. Alternativ oder zusätzlich kann die Halteanordnung insbesondere magnetisch wirken.

In weiterer Ausgestaltung der Erfindung weist die Halteanordnung ein an dem Gehäusebauteil angeordnetes Rastelement auf, das mittels einer Schwenkbewegung des Deckels von einem Rastabschnitt des Deckels überrastbar ist, wobei der Rastabschnitt das Rastelement in der Abdeckstellung lösbar formschlüssig hintergreift. Dies gestattet einen konstruktiv besonders einfachen und robusten Aufbau der Halteanordnung. Beim Überrasten des Rastelements führen der Rastabschnitt und das Rastelement eine axiale und damit entlang der Schwenkachse gerichtete Relativbewegung aus. Dies zusätzlich zu der aus der Schwenkbewegung resultierenden Relativbewegung. In weiterer Ausgestaltung der Erfindung sind das Rastelement und der Rastabschnitt mittels der Federanordnung entlang der Schwenkachse relativ zueinander federnd gelagert. Hierdurch wird die Funktion der Halteanordnung in besonders vorteilhafter Weise mittels der Federanordnung unterstützt. Die Halteanordnung wirkt hierbei mit der Federanordnung zusammen. Denn letztere drückt den Deckel seitlich in Richtung der Lagerstellung. Hierdurch sind das Rastelement und der Rastabschnitt mittelbar über die Federanordnung axial federnd gegeneinander gelagert. Dies ermöglicht insbesondere ein leichtgängiges Überrasten bei der Schwenkbewegung. Zudem unterstützt die Federanordnung den Formschluss zwischen dem Rastabschnitt und dem Rastelement.

In weiterer Ausgestaltung der Erfindung weist der Deckel zwei entlang der Schwenkachse voneinander beabstandet angeordnete Seitenwangen auf, die gegenüber einer Deckeloberseite abgewinkelt sind, wobei die Schwenklager und/oder die Federanordnung und/oder die Stützanordnung und/oder die Halteanordnung wenigstens abschnittsweise einstückig an wenigstens einer der Seitenwangen ausgebildet sind. Diese Ausgestaltung der Erfindung ermöglicht eine besonders vorteilhafte Reduktion der erforderlichen Anzahl an Bauteilen. Hierdurch wird ein besonders einfacher Aufbau erreicht. Zudem unterstützt die einstückige Ausbildung die Demontage und Montage, da die entsprechend einstückig ausgebildeten Bauteile und/oder Abschnitte quasi verliersicher an der wenigstens einen Seitenwange und damit an dem Deckel angeordnet sind. Die Seitenwangen liegen einander in Bezug auf die Schwenkachse axial gegenüber. In der Abdeckstellung übergreifen die Seitenwangen das Gehäusebauteil in seitlicher Richtung. In Kombination mit dieser Ausgestaltung der Erfindung ist eine Fertigung des Deckels als Kunststoffspritzgussbauteil besonders vorteilhaft.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt eine schematisch stark vereinfachte Darstellung eines Ausschnitts einer Ausgestaltung eines erfindungsgemäßen medizinischen Geräts zur extrakorporalen Blutbehandlung mit einem Gehäusebauteil und einem zeichnerisch ausgeblendeten Deckel,
- Fig. 2: in schematischer Perspektivdarstellung eine aus dem Stand der Technik bekannte Anordnung umfassend ein Gehäusebauteil, einen Deckel und eine Schwenklageranordnung zum Verschwenken des Deckels an dem Gehäusebauteil,
- Fig. 3: in einer - in Bezug auf die Zeichenebene der Fig. 1 - von oben nach unten gerichteten Blickrichtung das Gehäusebauteil des Geräts nach Fig. 1 mit dem daran gelagerten und eine Abdeckstellung einnehmenden Deckel,
- Fig. 4: in einer - in Bezug auf die Zeichenebene der Fig. 1 - teilweise abgeschnittenen Frontalansicht das Gehäusebauteil und der Deckel im Bereich einer Schwenkachse, wobei der Deckel ausgehend von der Abdeckstellung nach Fig. 3 in Richtung einer Freigabestellung (Fig. 5) verschwenkt ist,
- Fig. 5: das Gehäusebauteil und der Deckel in einer der Fig. 4 entsprechenden Darstellungsweise, wobei der Deckel die Freigabestellung einnimmt,
- Fig. 6: das Gehäusebauteil und der Deckel in einer abschnittsweisen Schnittdarstellung, wobei die Schnittebene durch die Schwenkachse verläuft, und
- Fig. 7: in abgeschnittener Perspektivdarstellung das Gehäusebauteil mit dem daran gelagerten Deckel, wobei der Deckel die Freigabestellung einnimmt und teiltransparent dargestellt ist.

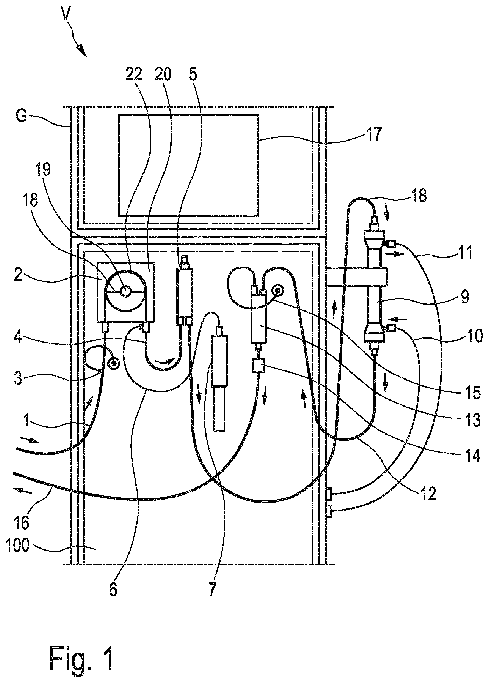

Gemäß Fig. 1 ist ein medizinisches Gerät V zur extrakorporalen Blutbehandlung abschnittsweise gezeigt und in Form eines Dialysegeräts gestaltet. Fig. 1 zeigt im Wesentlichen einen gesamten extrakorporalen Blutkreislauf des medizinischen Geräts V. Dieser weist eine arterielle Blutleitung 1 auf, mittels derer zu behandelndes Blut von einem nicht gezeigten Patienten zu einer Peristaltikpumpe 2 des medizinischen Geräts V geführt ist. In Bezug auf eine Förderrichtung des Bluts vor der Peristaltikpumpe 2 ist ein arterieller Druckabnehmer 3 vorgesehen. Mittels des arteriellen Druckabnehmers 3 ist der Druck in der arteriellen Blutleitung 1 vor der Peristaltikpumpe 2 erfassbar. Dieser Druck kann auch als niederdruckseitiger Druck bezeichnet werden. In Förderrichtung des Bluts nach der Peristaltikpumpe 2 - und somit hochdruckseitig - führt eine Hochdruckblutleitung 4 zu einer arteriellen Luftfalle 5. An einem Auslass der Peristaltikpumpe 2 ist vorliegend eine Zuleitung 6 angeordnet, die an einer Pumpe 7 angeschlossen ist. Über die Zuleitung 6 kann Zusatzstoff, beispielsweise Heparin zur Blutverdünnung, hinzudosiert werden. Ausgehend von der arteriellen Luftfalle 5 führt eine Leitung 8 das zu behandelnde Blut zu einem Dialysator 9, welchem eingangsseitig Dialysierflüssigkeit über eine Dialysierflüssigkeitszuleitung 10 zugeführt ist. In dem Dialysator 9 wird das Blut in einer bekannten Weise mittels der Dialysierflüssigkeit behandelt. Verbrauchte Dialysierflüssigkeit, die auch als Dialysat bezeichnet werden kann, wird über eine Dialysierflüssigkeitsableitung 11 von dem Dialysator 9 abgeleitet und einer nicht dargestellten Entsorgung oder Aufbereitung zugeführt. Das zu behandelnde Blut wird mittels einer Blutableitung 12 vom Dialysator 9 zu einer venösen Luftfalle 13 zur Abscheidung von Luft geleitet. Dieser nachgeschaltet ist ein Luftdetektor/Luftblasendetektor 14, der erkennt, ob sich für den Patienten gefährliche Luft in dem System befindet. An der venösen Luftfalle 13 ist ein venöser Druckabnehmer 15 vorgesehen, mittels dessen der venöse Druck erfassbar ist. Von der Luftfalle 13 über den Luftdetektor/Luftblasendetektor 14 wird das behandelte Blut über eine venöse Blutleitung 16 zurück zum Patienten geleitet. Zudem ist eine Steuer- und Überwachungseinrichtung 17 zur Steuerung und Überwachung des medizinischen Geräts V vorgesehen. Das medizinische Gerät V ist in einem Gehäuse G gekapselt, das eine Gehäusefront 100 aufweist, auf welcher insbesondere die Peristaltikpumpe 2 montiert ist.

Die Peristaltikpumpe 2 weist einen Rotor 18 und ein Pumpengehäuse 20 auf. Das Pumpengehäuse 20 bildet ein Gehäusebauteil des medizinischen Geräts V. In einem betriebsfertigen Zustand des medizinischen Geräts V ist das Pumpengehäuse 20 durch einen noch näher beschriebenen Deckel abgedeckt, der mittels einer ebenfalls noch näher beschriebenen Schwenklageranordnung schwenkbeweglich an dem Pumpengehäuse 20 gelagert ist. Sowohl der Deckel als auch die Schwenklageranordnung sind in Fig. 1 aus zeichnerischen Gründen nicht dargestellt.

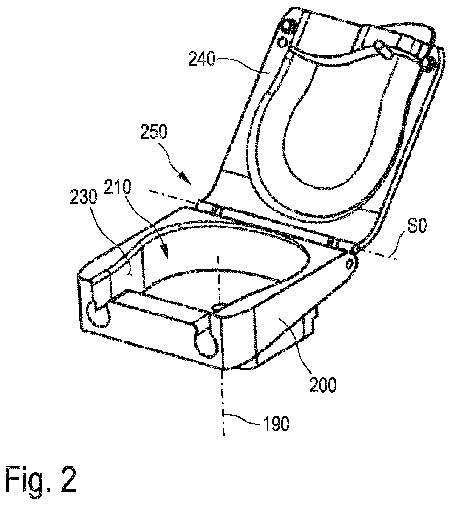

Zur Verdeutlichung des prinzipiellen Aufbaus und der Funktionsweise der Peristaltikpumpe 2 wird zunächst auf Fig. 2 Bezug genommen. Fig. 2 zeigt eine aus dem Stand der Technik bekannte Peristaltikpumpe mit einem Pumpengehäuse 200, einem Deckel 240 und einer Schwenklageranordnung 250. Das Pumpengehäuse 200 weist eine Aufnahmeaussparung 210 auf, die auch als Pumpenbett bezeichnet werden kann. In der Aufnahmeaussparung 210 ist ein anhand Fig. 2 nicht gezeigter Rotor angetrieben rotierbar aufgenommen. Die Aufnahmeaussparung 210 ist seitlich von einer bogenförmig um die Rotorachse 190 erstreckten und radial von dem Rotor beabstandeten Stützfläche 230 begrenzt.

Der Deckel 240 ist mittels der Schwenklageranordnung 250 um eine Schwenkachse S0 schwenkbeweglich an dem Pumpengehäuse 200 angelenkt. Der Deckel 240 nimmt in Fig. 2 einen geöffneten Zustand ein, in welchem die Aufnahmeaussparung 210 - in Bezug auf die Zeichenebene der Fig. 2 - nach oben freigegeben ist. Dieser Zustand kann auch als Freigabestellung bezeichnet werden. In einem geschlossenen Zustand deckt der Deckel 240 die Aufnahmeaussparung 210 nach oben ab. Dieser Zustand kann auch als Abdeckstellung bezeichnet werden.

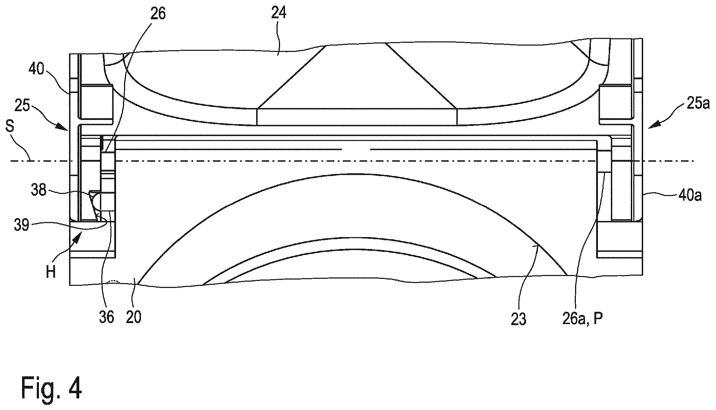

Die prinzipielle Funktionsweise der Peristaltikpumpe 2 zur Pumpförderung des Bluts zwischen der Niederdruck- und der Hochdruckseite ist identisch mit der Funktionsweise der anhand Fig. 2 gezeigten Peristaltikpumpe nach dem Stand der Technik. Hierzu ist eine elastisch deformierbare Schlauchleitung 22 in radialer Richtung zwischen dem Rotor 18 und der Stützfläche 23 (vgl. Fig. 4) des Pumpengehäuses 20 angeordnet. Die Schlauchleitung 22 ist an ihren gegenüberliegenden Stirnenden auf eine dem Fachmann bekannte Weise mit der arteriellen Blutleitung 1 und der Hochdruckblutleitung 4 fluidleitend verbunden. Zur Pumpförderung des Bluts wirkt der rotierende Rotor 18 auf die Schlauchleitung 22 ein, so dass diese abschnittsweise elastisch zwischen dem Rotor 18 und der Stützfläche 23 zusammengequetscht wird. Die hierbei entstehende Abquetschung der Schlauchleitung 22, die auch als Okklusion bezeichnet werden kann, wandert gleichsam mit dem rotierenden Rotor 18 um die Rotorachse 19, wodurch das Blut von der Niederdruck- auf die Hochdruckseite gefördert wird.

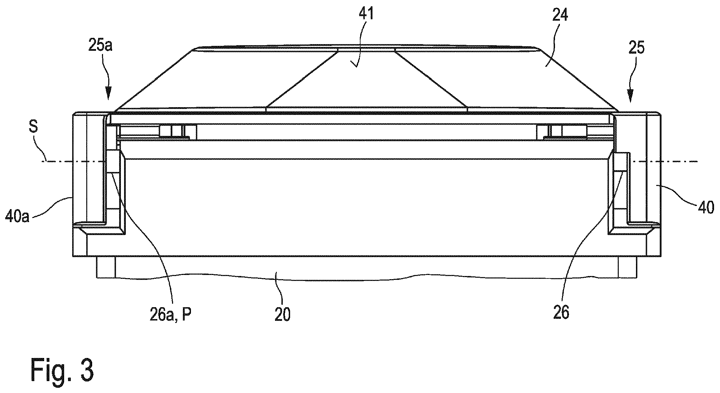

Bei der aus dem Stand der Technik bekannten Peristaltikpumpe nach Fig. 2 ist der Deckel 240 aufgrund der dort vorgesehenen Konstruktion der Schwenklageranordnung vergleichsweise aufwändig demontierbar und montierbar. Die anhand der Fig. 3 bis 7 im Detail ersichtliche erfindungsgemäße Gestaltung überwindet insbesondere diesen Nachteil.

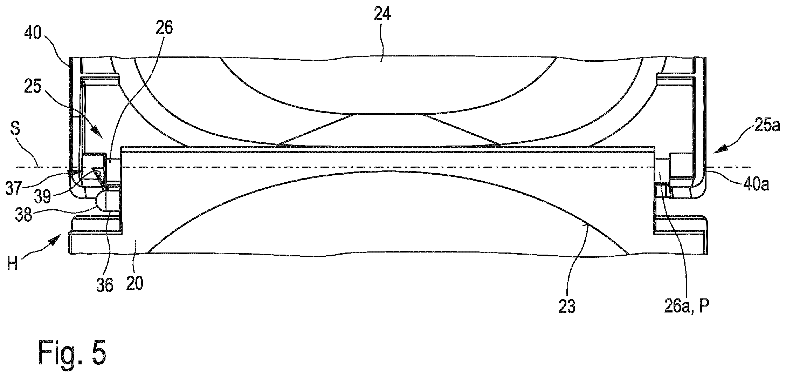

Dort ist gezeigt, dass der Deckel 24 mittels einer Schwenklageranordnung 25, 25a an dem Pumpengehäuse 20 und um eine Schwenkachse S relativ zu dem Pumpengehäuse 20 schwenkbeweglich gelagert ist. In Fig. 3 nimmt der Deckel 24 die Abdeckstellung ein. In dieser ist die Aufnahmeaussparung 21 des Pumpengehäuses 20 mittels des Deckels 24 abgedeckt. Ausgehend von der Abdeckstellung ist der Deckel 24 um die Schwenkachse S in Richtung einer Freigabestellung relativ zu dem Pumpengehäuse 20 verschwenkbar. In den Fig. 5, 6 sowie 7 nimmt der Deckel 24 die Freigabestellung ein. In dieser ist die Aufnahmeaussparung 21 - in Bezug auf die Zeichenebene der Fig. 3 - nach oben freigegeben. In dem anhand Fig. 1 gezeigten betriebsfertigen Zustand sind das Pumpengehäuse 20 und der Deckel 24 derart auf der Gehäusefront 100 montiert, dass die Schwenkachse S horizontal im Bereich einer nicht näher bezeichneten Oberkante des Pumpengehäuses 20 erstreckt ist.

Die Schwenklageranordnung 25, 25a weist bei der gezeigten Ausführungsform zwei Schwenklager auf, die entlang der Schwenkachse S voneinander beabstandet angeordnet sind. Die Schwenklager der Schwenklageranordnung 25, 25a werden nachfolgend auch als erstes Schwenklager 25 und zweites Schwenklager 25a bezeichnet.

Die Schwenklager 25, 25a weisen jeweils ein Lagerelement 26, 26a und eine Lagerelementaufnahme 27, 27a auf. Die Lagerelemente 26, 26a greifen jeweils in axialer Richtung in die jeweilige Lagerelementaufnahme 27, 27a ein und sind relativ zu dieser um die Schwenkachse S schwenkbeweglich. Die Lagerelemente 26, 26a werden nachfolgend auch als erstes Lagerelement 26 und zweites Lagerelement 26a bezeichnet. Entsprechendes gilt für die Lagerelementaufnahmen 27, 27a, die auch als erste Lagerelementaufnahme 27 und zweite Lagerelementaufnahme 27a bezeichnet werden.

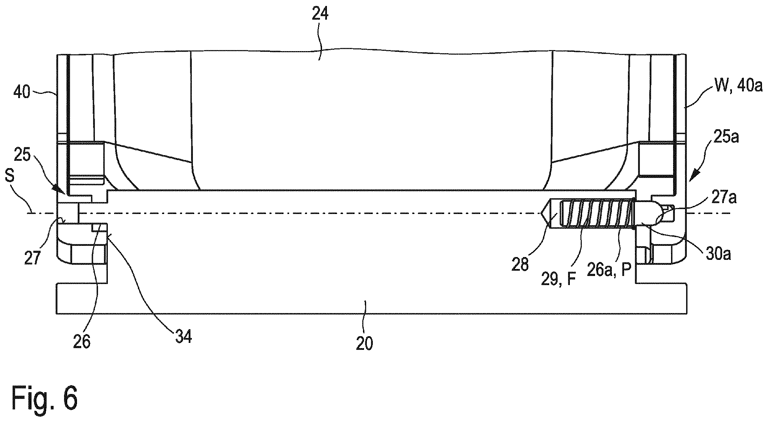

Insbesondere anhand Fig. 6 ist ersichtlich, dass die Lagerelemente 26, 26a und die Lagerelementaufnahmen 27, 27a nicht lediglich relativ zueinander um die Schwenkachse S schwenkbeweglich aneinandergelagert sind. Vielmehr ist gleichzeitig eine in axialer Richtung der Schwenkachse S begrenzte Linearbeweglichkeit zwischen den Lagerelementen 26, 26a und der jeweiligen Lagerelementaufnahme 27, 27a gewährleistet.

Hierzu ist bei der gezeigten Ausführungsform das zweite Lagerelement 26a in Form eines Lagerpins P gestaltet und begrenzt axialbeweglich in einer Aufnahmebohrung 28 des Pumpengehäuses 20 aufgenommen. Infolge dieser beweglichen Aufnahme des Lagerpins P kann der Deckel relativ zu dem Pumpengehäuse 20 in seitlicher Richtung, d.h. axial entlang der Schwenkachse S, bewegt werden. Die anhand der Fig. 3 bis 7 ersichtliche axiale Stellung des Deckels 24 in Bezug auf das Pumpengehäuse 20 kann auch als Lagerstellung bezeichnet werden. In dieser Lagerstellung sind beide Lagerelemente 26, 26a in Eingriff mit der jeweiligen Lagerelementaufnahme 27, 27a. Ausgehend von der Lagerstellung kann der Deckel 24 - in Bezug auf die Zeichenebenen der Fig. 4, 5, 6 - nach links gedrückt werden, bis die erste Lagerelementaufnahme 27 außer Eingriff mit dem ersten Lagerelement 26 gelangt. Der Deckel 24 befindet sich dann in einer Demontagestellung. Diese gestattet eine einfache manuelle Entnahme des Deckels 24 von dem Pumpengehäuse 20. Entsprechendes gilt sinngemäß für die Montage des Deckels 24.

Um eine ungewollte Demontage des Deckels 24 zu verhindern, ist zudem eine Federanordnung 29 vorgesehen, mittels derer der Deckel 24 entlang der Schwenkachse S federnd gelagert und in Richtung der Lagerstellung relativ zu dem Pumpengehäuse 20 elastisch vorgespannt ist.

Die Federanordnung 29 ist bei der gezeigten Ausführungsform als Federelement F ausgebildet. Das Federelement F ist in den Lagerpin P integriert, so dass dieser auch als Federpin P bezeichnet werden kann. Das Federelement F ist einends - jedenfalls mittelbar - an dem Pumpengehäuse 20 abgestützt und greift andernends an einer nicht näher bezeichneten Rückseite eines Kopfabschnitts 30a des Lagerpins P an. Der Kopfabschnitt 30a greift in die zweite Lagerelementaufnahme 27a ein.

Bei einer zeichnerisch nicht dargestellten Ausführungsform ist eine alternative Ausgestaltung der Federanordnung vorgesehen. Bei dieser ist der Lagerpin P axial unbeweglich an dem Pumpengehäuse 20 angeordnet und die federnde Lagerung und Vorspannung in axialer Richtung ist mittels einer elastisch formnachgiebigen Gestaltung des Deckels 24 im Bereich eines Wandabschnitts W erreicht. Durch die formnachgiebige Gestaltung des Wandabschnitts W kann der Deckel trotz axialfester Anordnung des Lagerpins P seitlich in Richtung der Demontagestellung gedrückt werden.

Das erste Lagerelement 26 ist bei der gezeigten Ausführungsform als Lagerzapfen gestaltet. Dementsprechend ist die erste Lagerelementaufnahme 27a als Zapfenaufnahme ausgebildet.

Weiter ist insbesondere anhand Fig. 6 ersichtlich, dass die Lagerelementaufnahmen 27, 27a jeweils einstückig an dem Deckel 24 ausgebildet sind. Die Lagerelemente 26, 26a sind demgegenüber an dem Pumpengehäuse 20 angeordnet, wobei das erste Lagerelement 26 einstückig an dem Pumpengehäuse 20 ausgebildet ist. Hierzu im Unterschied ist das zweite Lagerelement 26a bzw. der Lagerpin P als separates Bauteil gefertigt und durch Einstecken in die Aufnahmebohrung 28 mit dem Pumpengehäuse 20 zusammengefügt.

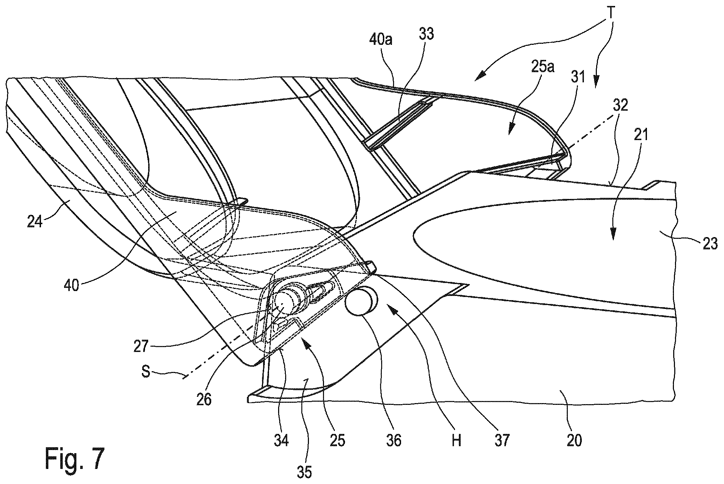

Um ein unbeabsichtigtes Entnehmen des Deckels 24 von dem Pumpengehäuse 20 zu verhindern, ist bei der gezeigten Ausführungsform eine zwischen dem Deckel 24 und dem Gehäusebauteil 20 ausgebildete Stützanordnung T vorgesehen (Fig. 7). Mittels der Stützanordnung T ist der Deckel 24 entgegen einer in die Demontagestellung gerichteten Linearbewegung an dem Pumpengehäuse 20 formschlüssig abstützbar. Dabei ist diese Abstützung vorliegend wenigstens in der Abdeckstellung bewirkt. Demgegenüber ist die Abstützung in einer definierten Schwenkstellung (Fig. 7) aufgehoben, wodurch der Deckel 24 - mangels formschlüssiger seitlicher Abstützung an dem Pumpengehäuse 20 - ausgehend von der Lager- in die Demontagestellung verschiebbar ist.

Die besagte definierte Schwenkstellung des Deckels 24 ist bei der gezeigten Ausführungsform die Freigabestellung. Bei einer nicht gezeigten Ausführungsform ist die definierte Schwenkstellung zur Entnahme des Deckels 24 eine ausgehend von der Freigabestellung weitergehend um die Schwenkachse S verschwenkte Schwenkstellung des Deckels 24.

Vorliegend weist die Stützanordnung ein an dem Deckel 24 angeordnetes Stützelement 31 auf, das zur Abstützung mit einem an dem Pumpengehäuse 20 angeordneten Stützabschnitt 32 formschlüssig zusammenwirkt. Der Stützabschnitt 32 ist durch eine seitliche Außenfläche des Pumpengehäuses 20 gebildet. Das Stützelement 31 ist vorliegend einstückig an dem Deckel 24 angeformt und liegt dem Stützabschnitt 32 in axialer Richtung der Schwenkachse S gegenüber. Dies jedenfalls in der Abdeckstellung. In der anhand Fig. 7 gezeigten Freigabestellung ist das Stützelement 31 zusammen mit dem Deckel 24 um die Schwenkachse S von dem Stützabschnitt 32 weggeschwenkt und somit nicht axial an demselben abstützbar. Mit anderen Worten ausgedrückt, blockiert das Stützelement 31 die axiale Beweglichkeit des Deckels 24 wenigstens in der Abdeckstellung derart, dass der Deckel 24 nicht ausgehend von der Lager- in die Demontagestellung verschieblich ist. Auch bei einer von der Abdeckstellung ausgehenden Schwenkbewegung des Deckels 24 bleibt das Stützelement 31 axial gegenüber dem Stützabschnitt 32 positioniert, so dass die Axialbeweglichkeit des Deckels auch dann blockiert ist. Erst in der definierten Schwenkstellung des Deckels 24, hier der Freigabestellung, ist das Stützelement 31 nicht mehr gegenüber dem Stützabschnitt 32 positioniert, so dass die Axialbeweglichkeit des Deckels in Richtung der Demontagestellung freigegeben ist.

Bei der gezeigten Ausführungsform weist die Stützanordnung T zudem ein weiteres Stützelement 33 auf. Das Stützelement 33 gewährleistet insbesondere in der Abdeckstellung eine möglichst flächige Abstützung an dem Stützabschnitt 32.

Weiter weist die Stützanordnung T vorliegend ein an dem Deckel angeordnetes Gegenstützelement 34 auf (Fig. 6, 7), das jedenfalls in der Abdeckstellung axial und entgegengesetzt zu dem Stützelement 32 an dem Pumpengehäuse 20 abgestützt ist. Hierfür weist das Pumpengehäuse 20 einen Gegenstützabschnitt 35 auf. Der Gegenstützabschnitt 35 ist in einer dem Stützabschnitt 32 entsprechenden Weise durch eine Seitenfläche des Pumpengehäuses 20 gebildet. Das Gegenstützelement 34 und der Gegenstützabschnitt 35 liegen einander in Axialrichtung der Schwenkachse S gegenüber und wirken einer - in Bezug auf die Zeichenebene der Fig. 6 - von links nach rechts gerichteten Axialbewegung des Deckels 24 entgegen. Hierdurch wird insbesondere ein ungewolltes Ausgreifen des Lagerpins P aus der zweiten Lagerelementaufnahme 27a verhindert. Bei der gezeigten Ausführungsform ist das Gegenstützelement 34 über den gesamten Schwenkbereich des Deckels 24 hinweg in axialer Richtung gegenüber dem Gegenstützabschnitt 35 positioniert. Mit anderen Worten ausgedrückt, ist der Deckel 24 nicht lediglich in der Abdeckstellung, sondern auch in allen weiteren Schwenkstellungen mittels des Gegenstützelements 34 an dem Gegenstützabschnitt 35 abstützbar.

In Bezug auf Fig. 1 ragt der Deckel 24 in der Freigabestellung schräg nach vorne oben von dem Pumpengehäuse 20 ab. Um den Deckel 24 entgegen der Einwirkung der Schwerkraft in der Freigabestellung zu halten, ist vorliegend eine Halteanordnung H vorgesehen. Die Halteanordnung H hält den Deckel entgegen einer in die Abdeckstellung gerichteten Schwenkbewegung in der Freigabestellung. Die Halteanordnung H ist zwischen dem Deckel 24 und dem Pumpengehäuse 20 ausgebildet. Vorliegend weist die Halteanordnung H ein an dem Pumpengehäuse 20 angeordnetes Rastelement 36 und einen an dem Deckel 24 ausgebildeten Rastabschnitt 37 auf. Das Rastelement 36 ragt in axialer Richtung der Schwenkachse S seitlich von dem Pumpengehäuse 20 ab und ist vorliegend im Bereich des Gegenstützabschnitts 35 angeordnet. Das Rastelement 36 ist an einem außenliegenden Stirnendbereich mit einer abgerundeten Rastkontur 38 versehen. Der Rastabschnitt 37 ist einstückig an dem Deckel 24 ausgebildet und weist eine angefaste Rastkontur 39 auf (Fig. 4). Mittels einer Schwenkbewegung des Deckels 24 ist das Rastelement 36 von dem Rastabschnitt 37 überrastbar. Hierbei gelangt zunächst die angefaste Rastkontur 39 zur gleitenden Anlage an der abgerundeten Rastkontur 38 des Rastelements 36. Dies ist in Fig. 4 gezeigt. Hierbei wird der Rastabschnitt 37 relativ zu dem Rastelement 36 in axialer Richtung nach außen gedrückt. Nach einem Überrasten des Rastelements hintergreift der Rastabschnitt 37 die abgerundete Rastkontur 38, so dass der Deckel 24 formschlüssig in der Freigabestellung gehalten ist.

Beim Überrasten des Rastelements 36 wirkt die Halteanordnung H in vorteilhafter Weise mit der Federanordnung 29 zusammen. Denn die zuvor beschriebene axiale Ausweichbewegung des Rastabschnitts 37 und damit des gesamten Deckels 24 wird durch die federnde Lagerung des Lagerpins P unterstützt. Hierdurch erfolgt das zuvor beschriebene Überrasten besonders leichtgängig und verschleißarm. Gleichzeitig unterstützt die federnde Lagerung bzw. die Vorspannung mittels der Federanordnung 29 das sichere Hintergreifen des Rastelements 36.

Weiter weist der Deckel 24 vorliegend zwei entlang der Schwenkachse S voneinander beabstandet angeordnete Seitenwangen 40, 40a auf, die auch als erste Seitenwange 40 und zweite Seitenwange 40a bezeichnet werden können. Die Seitenwangen 40, 40a sind gegenüber einer Deckeloberseite 41 des Deckels 24 abgewinkelt, wobei vorliegend ein Winkel von etwa 90° vorgesehen ist. Die Seitenwangen 40, 40a übergreifen das Pumpengehäuse 20 in der Abdeckstellung seitlich. Dabei sind die Seitenwangen 40, 40a axial gegenüberliegend dem Gegenstützabschnitt 35 bzw. dem Stützabschnitt 32 des Pumpengehäuses 20 angeordnet.

Die Lagerelementaufnahmen 27, 27a sind vorliegend im Bereich der Seitenwangen 40, 40a ausgebildet. Genauer ausgedrückt, ist die erste Lagerelementaufnahme 27 an der ersten Seitenwange 40 ausgebildet. Dabei ist die Lagerelementaufnahme 27 vorliegend in Form einer Durchgangsbohrung durch die erste Seitenwange 40 erstreckt. Die zweite Lagerelementaufnahme 27a ist im Bereich der zweiten Seitenwange 40a ausgebildet.

Zudem ist vorliegend auch die Stützanordnung T wenigstens abschnittsweise im Bereich der Seitenwangen 40, 40a ausgebildet (Fig. 7). Das Stützelement 31 und das weitere Stützelement 33 sind an einer innenliegenden Innenseite der zweiten Seitenwange 40a ausgebildet. Das Gegenstützelement 34 ist an einer der Innenseite der zweiten Seitenwange 40a gegenüberliegenden Innenseite der ersten Seitenwange 40 ausgebildet.

Zudem ist auch die Halteanordnung H wenigstens abschnittsweise im Bereich der Seitenwangen 40, 40a ausgebildet. Genauer ausgedrückt, ist der Rastabschnitt 37 an der Innenseite der ersten Seitenwange 40 ausgebildet.

Die vorbeschriebene, vorzugsweise einstückige, Ausbildung der entsprechenden Funktionsabschnitte der Schwenklageranordnung 25, 25a, der Stützanordnung T und der Halteanordnung H an den Seitenwangen 40, 40a, insbesondere deren Innenseiten, ist besonders vorteilhaft. Der Deckel 24 ist bei der gezeigten Ausführungsform als Kunststoffspritzgussbauteil gefertigt.

## Patentansprüche

1. Medizinisches Gerät (V) zur extrakorporalen Blutbehandlung, aufweisend
- eine Peristaltikpumpe (2) mit einem Pumpengehäuse (20), einen Deckel (24) und eine Schwenklageranordnung (25, 25a), mittels derer der Deckel (24) an dem Pumpengehäuse (20) und um eine Schwenkachse (S) relativ zu dem Pumpengehäuse (20) schwenkbeweglich gelagert ist,
- wobei der Deckel (24) wenigstens zwischen einer Abdeckstellung, in welcher der Deckel (24) das Pumpengehäuse (20) abdeckt, und einer Freigabestellung, in welcher der Deckel (24) das Pumpengehäuse (20) freigibt, schwenkbeweglich ist,
- und wobei die Schwenklageranordnung (25, 25a) wenigstens zwei entlang der Schwenkachse (S) voneinander beabstandet angeordnete Schwenklager (25, 25a) aufweist,
- **dadurch gekennzeichnet, dass** die Schwenklager (25, 25a) jeweils ein Lagerelement (26, 26a) und eine Lagerelementaufnahme (27, 27a) aufweisen,
- wobei die Lagerelemente (26, 26a) in die jeweilige Lagerelementaufnahme (27, 27a) eingreifen und relativ zu derselben um die Schwenkachse (S) schwenkbeweglich und entlang der Schwenkachse (S) begrenzt linearbeweglich sind,
- wodurch der Deckel (24) entlang der Schwenkachse (S) relativ zu dem Pumpengehäuse (20) linearbeweglich verlagerbar ist zwischen einer Demontagestellung, in welcher wenigstens eines der Lagerelemente (26, 26a) außer Eingriff mit der jeweiligen Lagerelementaufnahme (27, 27a) ist, und einer Lagerstellung, in welcher beide Lagerelemente (26, 26a) in Eingriff mit der jeweiligen Lagerelementaufnahme (27, 27a) sind,
- und wobei eine Federanordnung (29) vorgesehen ist, mittels derer der Deckel (24) entlang der Schwenkachse (S) federnd gelagert und in Richtung der Lagerstellung relativ zu dem Pumpengehäuse (20) elastisch vorgespannt ist.

2. Medizinisches Gerät (V) nach Anspruch 1, **dadurch gekennzeichnet, dass** die Federanordnung (29) wenigstens ein Federelement (F) aufweist, das einends an einem der Lagerelemente (26, 26a) angreift und andernends an dem Deckel (24) oder dem Pumpengehäuse (20) wenigstens mittelbar abgestützt ist, oder dass die Federanordnung durch einen elastisch formnachgiebigen Wandabschnitt (W) des Deckels (24) gebildet ist, an welchem Wandabschnitt (W) eines der Lagerelemente (26, 26a) oder eine der Lagerelementaufnahmen (27, 27a) ausgebildet ist.

3. Medizinisches Gerät (V) nach Anspruch 1 oder 2, **dadurch gekennzeichnet, dass** eine mit dem Deckel (24) und dem Pumpengehäuse (20) wirkverbundene Stützanordnung (T) vorgesehen ist, mittels derer der Deckel (24) entgegen einer in die Demontagestellung gerichteten Linearbewegung an dem Pumpengehäuse (20) formschlüssig abstützbar ist, wobei die Abstützung wenigstens in der Abdeckstellung bewirkt ist, und wobei die Abstützung in einer definierten Schwenkstellung des Deckels (24) für dessen Demontage aufgehoben ist.

4. Medizinisches Gerät (V) nach Anspruch 3, **dadurch gekennzeichnet, dass** die Stützanordnung (T) wenigstens ein an dem Deckel (24) angeordnetes Stützelement (31) aufweist, welches wenigstens in der Abdeckstellung entlang der Schwenkachse (S) an einem Stützabschnitt (32) des Pumpengehäuses (20) formschlüssig abgestützt ist, und welches in der definierten Schwenkstellung des Deckels (24) um die Schwenkachse (S) von dem Stützabschnitt (32) weggeschwenkt und somit nicht an demselben abstützbar ist.

5. Medizinisches Gerät (V) nach Anspruch 3 oder 4, **dadurch gekennzeichnet, dass** die definierte Schwenkstellung die Freigabestellung oder eine ausgehend von der Freigabestellung weitergehend um die Schwenkachse (S) verschwenkte Schwenkstellung des Deckels (24) ist.

6. Medizinisches Gerät (V) nach einem der Ansprüche 3 bis 5, **dadurch gekennzeichnet, dass** die Stützanordnung (T) wenigstens ein an dem Deckel (24) angeordnetes Gegenstützelement (34) aufweist, das wenigstens in der Abdeckstellung entlang der Schwenkachse (S) und entgegengesetzt zu dem Stützelement (31) an einem Gegenstützabschnitt (35) des Pumpengehäuses (20) formschlüssig abgestützt ist.

7. Medizinisches Gerät (V) nach einem der vorhergehenden Ansprüche, **dadurch gekennzeichnet, dass** eine mit dem Deckel (24) und dem Pumpengehäuse (20) wirkverbundene Halteanordnung (H) vorgesehen ist, mittels derer der Deckel (24) entgegen einer in die Abdeckstellung gerichteten Schwenkbewegung in der Freigabestellung gehalten ist.

8. Medizinisches Gerät (V) nach Anspruch 7, **dadurch gekennzeichnet, dass** die Halteanordnung (H) ein an dem Pumpengehäuse (20) angeordnetes Rastelement (36) aufweist, das mittels einer Schwenkbewegung des Deckels (24) von einem Rastabschnitt (37) des Deckels (24) überrastbar ist, wobei der Rastabschnitt (37) das Rastelement (36) in der Abdeckstellung lösbar formschlüssig hintergreift.

9. Medizinisches Gerät (V) nach Anspruch 8, **dadurch gekennzeichnet, dass** das Rastelement (36) und der Rastabschnitt (37) mittels der Federanordnung (29) entlang der Schwenkachse (S) relativ zueinander federnd gelagert sind.

10. Medizinisches Gerät (V) nach einem der vorhergehenden Ansprüche, **dadurch gekennzeichnet, dass** der Deckel (24) zwei entlang der Schwenkachse (S) voneinander beabstandet angeordnete Seitenwangen (40, 40a) aufweist, die gegenüber einer Deckeloberseite (41) abgewinkelt sind, wobei die Schwenklager (25, 25a) und/oder die Federanordnung (29) und/oder die Stützanordnung (T) und/oder die Halteanordnung (H) wenigstens abschnittsweise einstückig an wenigstens einer der Seitenwangen (40, 40a) ausgebildet sind.

## Claims

1. A medical appliance (V) for extracorporeal blood treatment, having
- a housing component (20), a cover (24) and a pivot bearing arrangement (25, 25a) by means of which the cover (24) is mounted on the housing component (20) and so as to be pivotable about a pivot axis (S) relative to the housing component (20),
- wherein the cover (24) is pivotable at least between a covering position, in which the cover (24) covers the housing component (20), and a release position, in which the cover (24) releases the housing component (20),
- and wherein the pivot bearing arrangement (25, 25a) has at least two pivot bearings (25, 25a) arranged spaced apart from one another along the pivot axis (S),
- **characterized in that** the pivot bearings (25, 25a) each have a bearing element (26, 26a) and a bearing element receptacle (27, 27a),
- wherein the bearing elements (26, 26a) engage in the respective bearing element receptacle (27, 27a) and are pivotable about the pivot axis (S) and linearly movable to a limited extent along the pivot axis (S), relative to said bearing element receptacle,
- as a result of which the cover (24) can be shifted in a linearly movable manner along the pivot axis (S) relative to the housing component (20) between a removal position, in which at least one of the bearing elements (26, 26a) is not in engagement with the respective bearing element receptacle (27, 27a), and a bearing position, in which the two bearing elements (26, 26a) are in engagement with the respective bearing element receptacle (27, 27a),
- and wherein a spring arrangement (29) is provided by means of which the cover (24) is mounted resiliently along the pivot axis (S) and is elastically pretensioned relative to the housing component (20) in the direction of the bearing position.

2. The medical appliance (V) as claimed in claim 1, **characterized in that** the spring arrangement (29) has at least one spring element (F) which at one end acts on one of the bearing elements (26, 26a) and at the other end is at least indirectly supported on the cover (24) or the housing component (20), or **in that** the spring arrangement is formed by an elastically dimensionally flexible wall portion (W) of the cover (24), on which wall portion (W) one of the bearing elements (26, 26a) or one of the bearing element receptacles (27, 27a) is formed.

3. The medical appliance (V) as claimed in claim 1 or 2, **characterized in that** a support arrangement (T) which is operatively connected to the cover (24) and to the housing component (20) is provided by means of which the cover (24) can be supported in a form-fitting manner on the housing component (20) counter to a linear movement directed toward the removal position, wherein the support is brought about at least in the covering position, and wherein the support is removed in a defined pivoted position of the cover (24) for removal thereof.

4. The medical appliance (V) as claimed in claim 3, **characterized in that** the support arrangement (T) has at least one support element (31) which is arranged on the cover (24) and, at least in the covering position, is supported in a form-fitting manner along the pivot axis (S) on a support portion (32) of the housing component (20), and which, in the defined pivoted position of the cover (24), is pivoted away from the support portion (32) about the pivot axis (S) and therefore cannot be supported on said support portion.

5. The medical appliance (V) as claimed in claim 3 or 4, **characterized in that** the defined pivoted position is the release position or a pivoted position of the cover (24) pivoted from the release position further about the pivot axis (S).

6. The medical appliance (V) as claimed in one of claims 3 to 5, **characterized in that** the support arrangement (T) has at least one mating support element (34) which is arranged on the cover (24) and, at least in the covering position, is supported in a form-fitting manner along the pivot axis (S) and counter to the support element (31) on a mating support portion (35) of the housing component (20).

7. The medical appliance (V) as claimed in one of the preceding claims, **characterized in that** a holding arrangement (H) which is operatively connected to the cover (24) and to the housing component (20) is provided by means of which the cover (24) is held in the release position counter to a pivoting movement directed into the covering position.

8. The medical appliance (V) as claimed in claim 7, **characterized in that** the holding arrangement (H) has a latching element (36) which is arranged on the housing component (20) and over which a latching portion (37) of the cover (24) can latch by means of a pivoting movement of the cover (24), wherein the latching portion (37) grips releasably in a form-fitting manner behind the latching element (36) in the covering position.

9. The medical appliance (V) as claimed in claim 8, **characterized in that** the latching element (36) and the latching portion (37) are mounted resiliently relative to each other along the pivot axis (S) by means of the spring arrangement (29).

10. The medical appliance (V) as claimed in one of the preceding claims, **characterized in that** the cover (24) has two side members (40, 40a) which are arranged spaced apart from each other along the pivot axis (S) and are angled in relation to a cover top side (41), wherein the pivot bearings (25, 25a) and/or the spring arrangement (29) and/or the support arrangement (T) and/or the holding arrangement (H) are formed integrally at least in sections on at least one of the side members (40, 40a).

## Revendications

1. Appareil médical (V) pour le traitement extracorporel du sang, comprenant
- une pompe péristaltique (2), comprenant un corps de pompe (20), un couvercle (24) et un dispositif formant palier de pivotement (25, 25a), au moyen duquel le couvercle (24) est monté sur le corps de pompe (20) et est apte à pivoter par rapport au corps de pompe (20) autour d'un axe de pivotement (S),
- ledit couvercle (24) étant apte à pivoter au moins entre une position de recouvrement, dans laquelle le couvercle (24) recouvre le corps de pompe (20), et une position de libération, dans laquelle le couvercle (24) libère le corps de pompe (20),
- et dans lequel le dispositif formant palier de pivotement (25, 25a) comprend au moins deux paliers de pivotement (25, 25a) agencés à distance l'un de l'autre le long de l'axe de pivotement (S),
- **caractérisé en ce que** les paliers de pivotement (25, 25a) comprennent chacun un élément de palier (26, 26a) et un logement (27, 27a) d'élément de palier,
- les éléments de palier (26, 26a) étant engagés dans le logement (27, 27a) d'élément de palier respectif et étant aptes à pivoter par rapport à celui-ci autour de l'axe de pivotement (S) et se déplacer de manière linéaire limitée le long de l'axe de pivotement (S),
- ce qui permet de déplacer le couvercle (24) selon l'axe de pivotement (S) de manière linéaire par rapport au corps de pompe (20) entre une position de démontage, dans laquelle au moins un des éléments de palier (26, 26a) est désengagé du logement (27, 27a) d'élément de palier correspondant, et une position de stockage, dans laquelle les deux éléments de palier (26, 26a) sont en engagement avec le logement (27, 27a) d'élément palier correspondant,
- et dans lequel un mécanisme à ressort (29) est prévu, au moyen duquel le couvercle (24) est monté de manière élastique le long de l'axe de pivotement (S) et qui est précontraint élastiquement en direction de la position de palier par rapport au corps de pompe (20).

2. Appareil médical (V) selon la revendication 1, **caractérisé en ce que** le mécanisme à ressort (29) comprend au moins un élément ressort (F) qui s'engage par une extrémité dans l'un des éléments de palier (26, 26a) et qui s'appuie par l'autre extrémité, au moins indirectement, sur le couvercle (24) ou sur le corps de pompe (20), ou **en ce que** le mécanisme à ressort est formé par une partie de paroi (W) du couvercle (24) qui est élastiquement déformable, partie de paroi (W) sur laquelle est formé l'un des éléments palier (26, 26a) ou l'un des logements (27, 27a) d'éléments de palier.

3. Appareil médical (V) selon la revendication 1 ou la revendication 2, **caractérisé en ce qu'**il est prévu un agencement de support (T) relié fonctionnellement au couvercle (24) et au corps de pompe (20), au moyen duquel le couvercle (24) est susceptible d'être soutenu par complémentarité de forme sur le corps de pompe (20) à l'encontre d'un mouvement linéaire dirigé vers la position de démontage, ledit support étant assuré au moins dans la position de recouvrement, et ledit support étant annulé dans une position de pivotement définie du couvercle (24), pour le démontage de celui-ci.

4. Appareil médical (V) selon la revendication 3, **caractérisé en ce que** l'agencement de support (T) comprend au moins un élément de support (31) agencé sur le couvercle (24), qui, au moins dans la position de recouvrement, est supporté par complémentarité de forme le long de l'axe de pivotement (S) sur une partie d'appui (32) du corps de pompe (20), et qui, dans la position de pivotement définie du couvercle (24), est éloigné par pivotement de la partie d'appui (32) autour de l'axe de pivotement (S) et ne peut donc pas être supporté par celle-ci.

5. Appareil médical (V) selon la revendication 3 ou la revendication 4, **caractérisé en ce que** la position de pivotement définie est la position de libération ou une position de pivotement du couvercle (24) qui est pivotée davantage autour de l'axe de pivotement (S) à partir de la position de libération.

6. Appareil médical (V) selon l'une des revendications 3 à 5, **caractérisé en ce que** l'agencement de support (T) comprend au moins un élément de contre-appui (34) agencé sur le couvercle (24), ledit élément étant soutenu par complémentarité de forme au moins dans la position de recouvrement le long de l'axe de pivotement (S) et à l'opposé de l'élément de support (31) sur une partie de contre-appui (35) du corps de pompe (20).

7. Appareil médical (V) selon l'une des revendications précédentes, **caractérisé en ce qu'**il est prévu un dispositif de maintien (H) relié fonctionnellement au couvercle (24) et au corps de pompe (20), au moyen duquel le couvercle (24) est maintenu en position de libération à l'encontre d'un mouvement de pivotement dirigé vers la position de recouvrement.

8. Appareil médical (V) selon la revendication **7, caractérisé en ce que** le dispositif de maintien (H) comprend un élément de verrouillage (36) agencé sur le corps de pompe (20), qui est apte à être verrouillé par une partie de verrouillage (37) du couvercle (24) au moyen d'un mouvement de pivotement du couvercle (24), la partie de verrouillage (37) s'engageant de manière amovible par complémentarité de forme dans l'élément de verrouillage (36) en position de recouvrement.

9. Appareil médical (V) selon la revendication 8, **caractérisé en ce que** l'élément de verrouillage (36) et la partie de verrouillage (37) sont montés de manière élastique l'un par rapport à l'autre le long de l'axe de pivotement (S) au moyen du dispositif à ressort (29).

10. Appareil médical (V) selon l'une des revendications précédentes, **caractérisé en ce que** le couvercle (24) comprend deux parois latérales (40, 40a) espacées l'une de l'autre le long de l'axe de pivotement (S), qui sont inclinées par rapport à une face supérieure de couvercle (41), les paliers pivotants (25, 25a) et/ou le mécanisme à ressort (29) et/ou l'agencement de support (T) et/ou le mécanisme de maintien (H) étant formés au moins partiellement d'un seul tenant sur au moins l'une des parois latérales (40, 40a).
